(19) Europäisches Patentamt
European Patent Office
Office européen des brevets

(11) EP 4 602 928 A1

(12) **EUROPEAN PATENT APPLICATION**

(43) Date of publication:
**20.08.2025 Bulletin 2025/34**

(21) Application number: **25157347.3**

(22) Date of filing: **12.02.2025**

(51) International Patent Classification (IPC):
***A23L 33/105*** *(2016.01)* ***A23L 33/21*** *(2016.01)*
***C12P 19/00*** *(2006.01)*

(52) Cooperative Patent Classification (CPC):
**A23L 33/21; A23L 33/105; C12P 19/00;**
C12P 2201/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.02.2024 LU 506397**

(71) Applicant: **SOREMARTEC S.A.**
**2633 Senningerberg (LU)**

(72) Inventors:
- **ARANCIO, Paolo**
  **L-5895 ALZINGEN (LU)**
- **CASERTANO, Melania**
  **6706 JR WAGENINGEN (NL)**
- **FOGLIANO, Vincenzo**
  **I-00166 ROMA (IT)**
- **MENNELLA, Ilario**
  **L-4276 ESCH SUR ALZETTE (LU)**
- **MONTEMURRO, Marco**
  **I-70126 BARI (IT)**

(74) Representative: **Rambelli, Paolo et al**
**Jacobacci & Partners S.p.A.**
**Corso Emilia 8**
**10152 Torino (IT)**

# (54) A PROCESS FOR UPCYCLING SPENT COFFEE GROUNDS INTO AN ANTIOXIDANT DIETARY FIBER

(57) A process for upcycling spent coffee grounds into an antioxidant dietary fiber comprising the steps of: a) microwave assisted extraction (MAE) of the spent coffee ground (SCG) material in water at a temperature up to 200 °C and for a time of at least 5 min, followed by b) enzymatic assisted extraction (EAE) of the spent coffee ground material extracted according to step a) in an aqueous solution comprising an enzyme having endo-beta-glucanase and/or an enzyme having cellulase activity and recovering the soluble matter. In a preferred embodiment, prior to the MAE extraction step, the SCG is subjected to a pretreatment selected from a pulsed electric field treatment or high-pressure extraction or both.

EP 4 602 928 A1

## Description

The present invention relates to an improved process for upcycling spent coffee grounds (SCG) into an antioxidant dietary fiber.

Coffee is a widely consumed and highly valued product worldwide which consumption is continuously increasing reaching around 10.1 billion kg in 2020, 1 billion more than in 2015 (International Coffee Organization, 2021). All steps of coffee processing generate a substantial amount of waste annually. The manufacture of instant coffee and the coffee brewing at domestic/retail level produce around 6 million tons of spent coffee ground (SCG) annually.

Recent research efforts have focused on studying the composition of SCG for their potential reuse, as suggested by the principle of circular economy. Polysaccharides, oligosaccharides, lipids, aliphatic acids, amino acids, proteins, alkaloids (e.g. caffeine, trigonellin), phenols, minerals, lignin, melanoidins, and volatile compounds are valuable constituents of SCG.

SCG possess functional qualities, due to the high quantity of fibers, tannins, and phenols, which results in high antioxidant potential. SCG fibers are largely insoluble and mostly composed of cellulose and hemicellulose, with a large amount of lignin also present.

Additionally, the SCG has a substantial amount of lipids and proteins with high biological value. SCG has been proposed as bulk material for several uses including adsorbers, fillers, additives for polymers production, supplement in animal feed, and soil fertilizers. However, SCG is a source of valuable bioactive compounds. Diterpene alcohol esters and phenolics represent the most prominent ones while condensed and hydrolysable tannins are additional active polyphenols found in SCG. Melanoidins are end-products of the Maillard reaction with high molecular weight and variable composition, usually incorporating also SCG phenolics. Melanoidins are associated with different biological activities including prebiotic, antioxidant, and antimicrobial. Dietary melanoidins bypass digestive processes in the gastrointestinal tract becoming substrates for the production of short chain fatty acids (SCFAs) by gut bacteria and then modulating the microbiota.

Besides melanoidins, mannooligosaccharides (MOS) are defined prebiotic due to their ability to influence the gut microbiota by promoting the proliferation of particular advantageous species. Therefore, considering the high concentration of fibers and melanoidins, SCG derived ingredients could be used as prebiotics or also as symbiotic to support the growth of probiotic.

Polar or intermediately polar solvents are usually employed for the extraction of these compounds from food by-products. Unfortunately, most of the coffee valuable molecules were extracted during brewing and the remaining in SCG are trapped within the abundant fiber network, therefore an extensive hydrolysis is required to extract and use them as a source of functional ingredients. Enzyme-assisted extraction (EAE) is very promising for achieving an efficient and cost-effective hydrolysis of plant matrices. Due to the high levels of lignin, cellulose, and hemicellulose, different enzymes including β-glucanases, cellulases, and hemicellulases can be used to solubilize SCG insoluble components.

Besides enzymes, physical methods can be used to hydrolyze SCG and especially microwave treatment (Microwave Assisted Extraction, MAE) was very effective to extract oligosaccharides and the phenolic fraction (Passes et al., 2013). Both for enzymatic and physical extraction methods the particles size is an important influencing factor for the extraction of phenolic compounds.

An objective of the present invention is to develop a novel process to increase the amount of soluble matter extracted from SCG to obtain an antioxidant dietary fiber suitable for human consumption.

A further object of the invention is to provide a process allowing to obtain, with improved yield, a prebiotic functional ingredient comprising oligosaccharides, preferably bound to polyphenols, that has a positive impact on the gut microbioma and that can trigger antiinflammatory pathways at the gut epithelium level.

A further object of the invention is to provide a process that allows to obtain a prebiotic functional ingredient with improved antioxidant properties.

## Summary of the invention

The subject matter of the invention is defined by the appended claims.

The process of the invention combines the step of microwave assisted extraction (MAE) of the raw spent coffee ground material with the step of enzymatic assisted extraction (EAE) carried out on the spent coffee ground material which is the extracted residue of the MAE step.

According to the invention, it has been found that by combining said two steps, the overall amount of valuable soluble matter is enhanced, since it is higher than the overall amount that is obtained by subjecting the same raw SCG to MAE and separately to EAE, under the same process conditions adopted for each step in the combined process. Moreover it was found that, by carrying out the extraction process in the sequence MAE followed by EAE, the amount of the obtained soluble matter is significantly enhanced in comparison with the amount obtained with the opposite sequence.

The process of the invention is further described by the following detailed description and working examples, with reference to the appended drawings.

**Brief description of the drawings**

In the following examples and in the drawings:

SCG-C indicates the amount of soluble matter (SM) released in water by SCG after cryogenic milling;
SCG-M indicates the amount of soluble matter (SM) released in water by SCG after MAE;
SCG-E indicates the amount of soluble matter (SM) released in water by SCG after EAE;
SCG-CE indicates the amount of soluble matter (SM) released in water by SCG after cryogenic milling and EAE;
SCG-ME indicates the amount of soluble matter (SM) released in water by SCG after MAE and EAE.

In the annexed drawings:

Figure 1 describes the step-by-step process employed for the upcycling of SCG into a prebiotic functional ingredient. The grinding and defatting steps in figure 1 are optional;
Figure 2 is a pie chart showing the composition of the raw SCG used in the following examples;
Figure 3 is a schematic flow chart showing a mass balance of the process of the invention;
Figure 4 is a histogram showing the quantification of galactose, fucose, arabinose, galactose, glucose, mannose, and fructose in SCG after the combination of microwave extraction and enzymatic treatment (SCG-ME), by extraction of a raw SCG from an industrial coffee brewing process;
Figure 5 is a histogram showing the quantification of galactose, fucose, arabinose, glucose, mannose, rhamnose, and xylose in SCG from coffee houses, before any treatment (SCG), after cryogenic milling (SCG-C), MAE (SCG-M), EAE (SCG-E), combination of cryogenic milling and EAE (SCG-CE), and combination of MAE and EAE (SCG-ME).
Figure 6 and Table 2 show the composition and characteristics of the functional ingredient obtained after the step of spray-drying.
Figure 7 is a histogram showing the amount of soluble fraction released in water in SCG before any treatment (SCG), after cryogenic milling (SCG-C), MAE (SCG-M), EAE (SCG-E), combination of cryogenic milling and enzymatic treatment (SCG-CE), and combination of MAE and EAE (SCG-ME);
Figure 8. is a histogram showing the antioxidant properties of an SCG from coffee houses, before any treatment (SCG), after cryogenic milling (SCG-C), MAE (SCG-M), EAE (SCG-E), combination of cryogenic milling and EAE (SCG-CE), and combination of MAE and EAE (SCG-ME) evaluated by DPPH, ABTS, FRAP assays.

**Detailed description**

**Analytical methods**

The raw SCG used in the process of the invention may originate from different sources; it may be a waste from coffee machine (from domestic appliances or from coffee houses) or from industrial coffee brewing plants.
Figure 2 shows the proximate composition of a raw SCG, after thermal stabilization, from an industrial coffee brewing process.
Table 1 shows the proximate composition of a raw SCG, after thermal stabilization, from a coffee house.

Table 1. Proximal composition of spent coffee grounds after thermal stabilization process.

| | g/100g |
|---|---|
| Fat | 14.99 ± 0.02 |
| Protein | 12.83 ± 0.15 |
| Insoluble fiber | 50.24 ± 2.27 |
| Soluble fiber | 5.46 ± 0.36 |
| Carbohydrates | 6.40 ± 2.86 |
| Ash | 1.05 ± 0.05 |
| Moisture | 9.03 ± 0.36 |

Proximate analyses were conducted to determine the percentage of moisture, ash, protein, fat, soluble and insoluble fiber, and carbohydrate. In details:

- moisture was determined according to AACC method n. 44-15.02;
- the ash content was determined according to AACC method n. 08-01;
- the protein content was evaluated by the Dumas method using a Flash EA 1112 NC analyser (Thermo Fisher Scientific Inc., Waltman, USA) following the manufacturer's protocol, the conversion factor 6.25, usually applied to SCG, was used (Massaya et al., 2019).;
- the fat content was determined by Soxhlet extraction. 3-5 grams of sample was weighed in an extraction thimble. 200 ml Petroleum ether 40-60°C was added to a flat bottom flask with boiling chips. All weights were registered carefully for the fat determination. The flat bottom flasks were connected to the extractors containing the thimbles with sample and coolers and were placed on a heating block. The heating was turned on and the extraction was performed for 5 hours. After cooling down, the flat bottom flasks were evaporated using Rotation Evaporators of Büchi. The thimbles and the evaporated flat bottom flasks were left overnight in a fume hood to let the leftover petroleum ether evaporate. The dry flat bottom flasks were weighed, and the fat content was determined using the amount of extracted fat compared to the amount of sample used.
- Soluble and insoluble fibers were determined according to AACC Method n. 32-07.01 by using K-TDFR-200A Megazyme kit (Megazyme, Ireland).

Particularly, to determine the amount of Soluble Dietary Fiber (SDF), 4 vols of preheated EtOH (95%) 60°C was added to the sample in a 50ml Greiner tube. The precipitate was allowed to form at room temperature for 60 minutes. The samples were centrifuged, and the supernatant was discarded. The pellet was transferred to a 15 ml Greiner tube with a known weight. The tube was closed off with cotton papers and the pellet was freeze-dried.

The analysis was done in duplicate and Equation 2 was used to determine the SDF, taking into account the dilution used during extraction.

$$SDF: \frac{dry\ sample\ weight}{initial\ sample\ weight} * 100\% \qquad \text{Equation 2}$$

- The carbohydrates were determined by difference (Nitisewojo, 1995).
- The total amount of dry matter present in the soluble fraction (SDM) was determined by taking ±2 ml of soluble fraction (supernatant after centrifugation) and transferring it onto an aluminum dish. The weights of the sample and aluminum dish were noted carefully. The dish was dried overnight in an oven at 100°C until constant weight. The dry weight of the samples was determined, and the soluble solid content was determined and expressed as mg SDM/g SCG with Equation 1, taking into consideration the dilution used for the extraction process.

$$SDM = \frac{dry\ sample\ weight}{wet\ sample\ weight} \qquad \text{Equation 1}$$

- The total phenolics content (TPC) was determined based on the Folin-Ciocalteu method of Singleton and Rossi (Singleton & Rossi, 1965). Folin-Ciocalteu is a brightly yellow coloured reagent. By measuring the absorbance at 760 nm, the number of hydroxyl groups present in the sample can be determined. These hydroxyl groups are directly linked to the presence of phenols.

20% NaOH was made by solubilizing 50g $NaCO_3$ in 250 ml MiliQ water. A gallic acid calibration curve was made with the following concentrations: 0.025, 0.05, 0.1, 0.15 and 0.25 mg/ml. 0.2ml sample, standard or blank were mixed with 1.88 ml MiliQ water, 0.11 ml Folin-Ciocalteu reagent and 0.31 ml $NaCO_3$. The tubes were shaken carefully and left in a dark environment for 60 minutes at room temperature. Afterwards, the absorbances were determined using a spectrophotometer (Cary 60 spectrophotometer (Agilant Technologies Inc.)) and the gallic acid equivalent (GAE) was calculated by comparing the calibration with the sample absorbances.

In the analysed SCG from an industrial coffee brewing process (fig 2), the highest concentration of insoluble fiber (50.83g /100g of SCG dry matter) and the lowest concentration of soluble fiber (4.00 g/100g of SCG dry matter) were found. The fat content of the analysed raw material was of about 20% wt (dry matter), whereas the fat content from a coffee house (table 1) was of only about 15%.

**Antioxidant activity**

The antioxidant activity of SCG extracts was evaluated by DPPH, ABTS, FRAP assays (Figure 8).

For the DPPH analysis, the method described by Brand-Williams et al. (1995) was used, with some modification, by preparing a 0,1 mM solution of 2,2-diphenyl-1-picrylhydrazyl (DPPH) in methanol. 50μl of sample, standard or blank, was added to 1.95ml DPPH solution. The samples were vortexed and kept at room temperature for 60 minutes in a dark environment. After incubation, the absorbances were measured at 515 nm.

**Process description**

In a preferred optional embodiment, before the extraction steps, the raw SCG is ground to reduce the particle size, preferably to a particle size having a D[4,3] below 1mm, preferably below 100 microns.

Since the raw SCG usually contains water (up to 70% wt), it is preferable to dry the raw SCG, before milling to a water content of about 10% wt or lower.

For the purpose of grinding, any grinding method known in the art that can ensure a particle size below 1000 - 900 microns can be used; however particularly preferred are grinding with a ball mill or more preferably cryogenic grinding.

As noted above, depending on the source of SCG, the amount of fat may vary in a relatively wide range. Whereas the fat content was not found to have a strong influence on the yield in soluble matter of the process, in a preferred embodiment of the invention and in order to improve the organoleptic properties of the target prebiotic functional ingredient and reduce oxidation phenomena, it is recommended to apply a defatting step prior to extraction, to reduce the fat content to below 5%, preferably below 2%.

Defatting may be accomplished by methods known in the art, but Soxhlet type extraction, preferably with petroleum ether, is preferred. The time of extraction is in the range of from 2 hours to 16 hours.

Also, supercritical carbon dioxide extraction can be used.

According to the invention, microwave assisted extraction (MAE) is used with the aim to increase the soluble fraction. Microwave-assisted extraction is a process of using microwave energy to heat solvents in contact with a sample in order to partition analytes from the sample matrix into the solvent. The ability to rapidly heat the sample solvent mixture is inherent to MAE and the main advantage of this technique. By using closed vessels, the extraction can be performed at elevated temperatures accelerating the mass transfer of target compounds from the sample matrix. Preferably, monowave extraction is used.

Water is the preferred solvent for MAE, preferably in a mass ratio (SCG /water) of from 1:3 to 1: 4. MAE could be applied for a time range of 2 to 10 min, preferably from 5 to 10 min, at a temperature of about 200°C.

According to the process of the invention the MAE extracted SCG slurry is then directly subjected to enzymatic assisted extraction (EAE). Alternatively, the soluble matter may be separated and recovered from the solid extracted SCG phase which is then subjected to EAE.

The EAE step b) is carried out in an aqueous solution comprising an enzyme having endo-beta-glucanase and/or cellulase activity.

Also, enzymes having mannanase, xylanase and /or hemicellulose activities or side activities may be used or added to the enzymes having endo-beta-glucanase and/or cellulase activity. The concentration of the enzymes is preferably: from 33 to 66 micro-L/g of SCG for the enzyme having endo-beta-glucanase activity of 300 amyloglucosidase Units/ml, and from 11 to 40 micro-L/g SCG for the enzyme with cellulase activity of 700 endo-glucanase unit/g.

The extraction is preferably carried out in temperature range of from 45 to 65°C, at a pH of from 4. 5 to 6 for a time of from 3 to 15 hours.

Preferred commercial enzymes that can be used include Viscozyme ™ L and Celluclast ™ 1.5L.

Viscozyme L is a broad-spectrum food grade enzyme that hydrolyses plant tissue (Novozymes, 2023); The key enzyme activity of Viscozyme is endo-β-glucanase, but also has side activity of xylanase, cellulase and hemicellulase. Celluclast 1.5L is a food grade enzyme, having cellulase activity that breaks down fibrous plant tissue and increases the extraction yield of vegetable products (Novozymes, 2023).

The enzymatic reaction may be stopped by raising the temperature e.g. to 90°C and maintaining this temperature for e.g. 10 min.

Following the EAE the supernatant comprising the soluble matter is collected after centrifugation.

Drying of the soluble matter to finally obtain the antioxidant dietary fiber may be accomplished by methods known in the art, including freeze-drying and drum-drying. Preferably the antioxidant dietary fiber is obtained by spray drying.

In a further embodiment of the invention the raw SCG prior to the MAE extraction may be subjected to a pretreatment selected from a pulsed electric field treatment (PEF) or high-pressure extraction (HPE) or both. PEF and HPE can be performed preferably as pretreatments followed by grinding and defatting, in between or after these steps, but necessarily before the MAE extraction.

PEF is a non-thermal approach that improves mass transfer by causing cell membranes to become permeable through electroporation under the influence of brief (ranging from nanoseconds to milliseconds) and moderately intense electric pulses. With low consumption of energy and minimal environmental impact, this method can hasten the release of intracellular chemicals and boost the rates and yields of various components from vegetal matrices. Preferably PEF could be used under the following parameters: field strength 1.5 kV/cm; 20 hz; pulse width of 2 μs; conductivity of 0,5 mS/cm and stating temperature of 10-12°C. Number of pulses might vary from 1000 to 10000 with a solid /liquid ratio % range of 30-42,5%.

HPE operates at low temperatures (usually up to 60 °C) and high pressures (usually 100-600 MPa) to extract the compounds quickly, requiring low quantities of organic solvents and at the same time provides retrievals similar to other extraction technologies. As the above-mentioned PEF, it is a good alternative to traditional heat treatment because it decreases the extraction time, and solvent consumption, and increases the extraction yield.

For the objective of the present patent, HPE may be also used as pretreatment. In this scenario, SCG samples might be suspended in deionized water in a ratio 2/3 v/v and then packaged in plastic bags under vacuum. Pressure and time conditions might vary from 200 MPa to 600 MPa and 1 min to 5 min, respectively.

## Example 1

### Cryogenic grinding (optional) and particle size evaluation

SCG were finely ground by using the cryo-miller 6875D Freezer/Mill® (Spex Sample Prep, United States). In details, 20 g of SCG were milled three times for 5 minutes with a setting of 10 cycles per second (cps).

The particle size distribution was analyzed using the Mastersizer 3000 (Malvern Instruments, Worcestershire UK) equipped with Aero S dispersion accessory. The particle refractive index 1.530 was optimized and used for coffee powder. Values of D[3,2] (surface weight mean diameter), D[4,3] (volume-weight mean diameter) and D(50) (volume median diameter) were obtained. Moreover, the parameters D(10), and D(90) were also calculated and indicate the portion of particles (10 and 90%, respectively) with diameter below the value of each parameter.

The particle size distribution of the dried SCG was characterized by the following parameters: D[3,2] of 102.8 $\pm$ 2.6 $\mu$m, D[4,3] of 372.4 $\pm$ 7.2 $\mu$m, D(10) of 34.2 $\pm$ 0.5 $\mu$m, D(50) of 292.3 $\pm$ 4.9 $\mu$m, and D(90) of 638 $\pm$ 11.7 $\mu$m. The cryo-milling process led to a significant decrease in SCG particle size. Indeed, SCG-C was characterized by D[3,2] of 33.7 $\pm$ 0.6 $\mu$m, D[4,3] of 93.6 $\pm$ 3.3 $\mu$m, D(10) of 15.0 $\pm$ 0.2 $\mu$m, D(50) of 44.4 $\pm$ 0.9 $\mu$m, and D(90) of 243.1 $\pm$ 8.5 $\mu$m. Therefore, the highest reduction was found in D(50), resulting in a particle size reduction of 85% in 50% of the particles of SCG-C.

In a previous study made on SCG (taken from a coffee machine, Iberital IB7) the cryogenic milling led to a total extracted amount of soluble matter of 40 %.

## Example 2: defatting step d) (optional)

The defatting process is preferably performed when the amount of fat in the raw SCG extracted by Soxhlet 'is high (e.g. >15%).

3 grams of SCG are weighed in thimbles and placed in extractors connected with a flat flask filled with 200 ml of petroleum ether put on a heater. After 5 hours of extraction, the thimbles containing the SCG defatted are left overnight to dry.

## Example 3: Microwave assisted extraction step a)

SCG were treated by using Monowave 400 (Anton Paar, Austria)

The Anton Paar Monowave product line is a series of high performance monomode microwave reactors designed for small to medium-scale microwave sinthesys (installed microwave power: 850 W (single magnetron).

4 g of SCG and 12 g of demineralized water were added to a glass vial G30 wide neck. The treatment by using Monowave 400 was performed using the stirrer speed of 300 rpm and the maximum power of 850 W to reach 200 °C and maintaining the temperature of 200°C for 10 min and then decreasing the temperature to 70 °C. The time needed to reach the set temperature was 145 seconds and the maximum pressure of 30 bar was reached. The total time needed for the treatment including the cool down to 55 °C was approximately 27 minutes.

In order to optimize the time of extraction, the soluble matter was investigated in the SCG extracted with only monowave by varying the extraction time from 10 to 2 minutes. There were no significant differences between 10 min and 5 min but there were between 10 min and 2 min; thus, the preferred extraction time is from 5 to 10 minutes, most preferably 5 minutes.

## Example 4: Enzymatic assisted extraction step b)

Enzymatic assisted extraction was carried out sequentially on the slurry resulting from the MAE step of example 3 or independently on the raw cryo milled SCG of example 1, using the commercial enzymes Viscozyme™ and Celluclast™, with the following process conditions:

| | |
|---|---|
| μL enzyme / g SCG: | 66 μL/g Viscozyme |
| | 22.6 μL/g Celluclast |
| and | 33 μL/g Viscozyme |
| | 11.2 μL/g Celluclast |

Temperature: 55°C
pH: 5.95
time from 4 to 14 hours.

No significant differences were observed in terms of soluble matter between the above two amounts of the enzymes and treatment times: soluble matter about 30- 33%

**Example 5: spray drying step**

A Büchi mini spray dryer (Büchi Laboratoriums-Technik, Zwitzerland) was used. A minimum of 100 ml supernatant extracts was used for spray-drying. A peristaltic pump pumped the SCG supernatant to the atomizer. The atomization was performed using a fluid nozzle. Compressed air from in-house supply was used. The compressed air was regulated by a flow meter, and cooling water was circulated through a jacked around the nozzle. Inlet drying air was set at 120°C, aspiration at 95%, and the pump at 20%. This inlet drying air, after passing through an electrical heater, flowed concurrently with the spray through the main chamber. Dried powder samples were collected from the base of the cyclone.

Spray drying of the supernatant obtained after centrifuging the MAE and EAE extraction product from example 4, was carried out under the following conditions:

Inlet temperature: 120 -160°C
Aspiration rate: 95%
Pump: 20 %

**Example 6**

Figure 3 shows the lay out of the process of the invention according to a specific working example.

3 g of dried SCG from an industrial coffee brewing process were diluted in water at a 1:4 dilution (12 ml water) and subjected sequentially to MAE and EAE according to the preferred conditions defined in the previous examples. The enzymatic reaction was then stopped by raising the temperature to 90° for 10 min.; 24 ml of water were added.

The dry matter in 36 ml was 0.95 g with a yield of solubilization of 31.7 %. The extracted slurry was centrifuged to provide about 23.5 ml of supernatant out of 36 ml (yield 65.4%). The supernatant was spray dried to provide 0.43 g of the desired functional ingredient; overall yield of the process: 14.3%.

**Example 7**

Raw SCG from an industrial coffee brewing plant was used, having a fat content of about 20%.

**Grinding process: step c)**

The grinding process was performed to reduce the particle size of the sample as it is supposed to facilitate the following steps. SCG was treated by using a household grinder Bosch TSM6A013B in which 30 g of SCG were ground for 5 minutes with a power rating of 180 W.

**Defatting process: step d)**

The defatting was performed for the high amount of fat previously detected on the SCG. The defatting was performed in the beaker in which 50 gr of SCG are stirred in 200 ml of petroleum ether at 300 rpm for 16 hours.

**Microwave-assisted extraction: step a)**

The defatted SCG was treated according to the process described in Example 3. All the samples were frozen at -20°C before subsequent treatment or analysis.

**Enzymatic extraction: step b)**

The enzymatic extraction was sequentially performed in a ratio 1:4, SCG:water. Viscozyme®L consists mainly in endo-beta-glucanase that hydrolyzes (1,3)- or (1,4)-linkages in β-D-glucans (100 FBG/g) and Celluclast®1.5 L providing cellulase that hydrolyzes (1,4)-β-D-glucosidic linkages in cellulose and other β-D-glucans (700 EGU/g) where provided by Novozymes (Denmark). The suggested dosages were 200-400 mL and 100-200 mL per ton of vegetable, respectively, the optimal pH range 3.3-5.5 and 4.0-6.0, respectively, and optimal temperature 40-50 and 50-60, respectively. The concentrations used based on the previous experiment were 33 μl/g Viscozyme and 11.2 μl/g Celluclast. The time of incubation was performed at 4 hours. The temperature was set at 55°C and the pH at 5.95. The enzymatic reaction was stopped at 90°C for 10 min. The supernatant was collected after centrifugation at 4500 rpm for 10 minutes.

**Spray-drying step**

A Büchi mini spray dryer (Büchi Laboratoriums-Technik, Zwitzerland) was used. A minimum of 100 ml supernatant extracts was used for spray-drying. A peristaltic pump pumped the SCG supernatant to the atomizer. The atomization was performed using a fluid nozzle. Compressed air from in-house supply was used. The compressed air was regulated by a flow meter, and cooling water was circulated through a jacked around the nozzle. Inlet drying air was set at 120°C, aspiration at 95%, and the pump at 20%. This inlet drying air, after passing through an electrical heater, flowed concurrently with the spray through the main chamber. Dried powder samples were collected from the base of the cyclone.

**Example 8**

Raw SCG from an industrial coffee brewing process was used, having a fat content of about 20%.

**Grinding step c)**

The grinding process was performed to reduce the particle size of the sample as it is supposed to facilitate the following steps. SCG was treated by using a) Cryo-miller 6875D Freezer/Mill, for example, 20 g of SCG were milled three times for 5 minutes with a setting of 10 cycles for second (cps) b) Household grinder Bosh TSM6A013B in which 30 g of SCG were ground for 5 minutes with a power rating of 180 W; c) Ball mill to mill 30 g of SCG for 30 min at a speed of 100 rpm.

**Defatting step d)**

The defatting was performed for the high amount of fat previously detected. Different methods have been performed such as a) Soxhlet in which 3 g of SCG are weighed in thimbles and placed in the extractor connected with a flat flask filled with 200 ml of petroleum ether put on heater. After 5 hours of extraction, the thimbles containing the SCG defatted are left overnight to dry; b) and c) the defatting was performed without heat treatment, but the sample is only stirred with the solvent such as petroleum ether. In b) 10 gr of SCG are mixed with 225 ml of petroleum ether and stirred at 300 rpm and time 30 min; in c) 50 gr of SCG are stirred in 200 ml of petroleum ether and stirred at 300 rpm for 16 hours.

**Microwave -assisted extraction step a)**

The defatted SCG was treated by using the Monowave 400 (Anton Paar, Austria) according to Example 3. All the samples were frozen at -20°C before subsequent treatment or analysis.

**Enzymatic extraction step b)**

The enzymatic extraction was performed as described in Example 7. in a ratio 1:4, SCG: water using. Viscozyme®L and Celluclast®1.5 LThe concentrations used were a) 66μl/g Viscozyme and 22.6μl/g Celluclast; and b) 33μl/g Viscozyme and 11.2 μl/g Celluclast. The time of incubation was performed at a) 14 hours and b) 4 hours. The temperature has been set at 55°C and the pH at 5.95. The enzymatic reaction was stopped at 90°C for 10 min. The supernatant was collected after centrifugation at 4500 rpm for 10 minutes.

**Spray-drying step**

A Büchi mini spray dyer (Büchii Laboratoriums-Technik, Zwitzerland) was used. A minimum of 100 ml supernatant extracts was used for spray-drying. The effect of the addition of an additive was investigated by adding 5% maltodextrin to the supernatant extract and stirring the solution until the maltodextrin was dissolved before spray-drying. A peristaltic pump pumped the SCG supernatant to the atomizer. The atomization was performed using a fluid nozzle. Compressed air from in-house supply was used. The compressed air was regulated by a flow meter, and cooling water was circulated through a jacked around the nozzle. Inlet drying air was set on 120°C, aspiration at 95% and the pump at 20%. This inlet drying air, after passing through an electrical heater, flowed concurrently with the spray through the main chamber. Dried powder samples were collected from the base of the cyclone.

**Results**

The antioxidant activity measured by DPPH and ABTS was respectively 33 mM Trolox Eq/g and 14 mg Trolox eq/g. The total polyphenols content was 28.46 mg gallic acid eq/g.

**Example 9**

Raw SCG from an industrial coffee brewing process was used, having a fat content of about 20%.

**Pulsed electric field (PEF)**

The spent coffee ground was pretreated by a pulsed electric field (PEF). The treatment was performed by mixing the sample with water 40.5% MS at 1.5 kV and 10000 pulses.

**Grinding step c)**

The grinding process has been performed to reduce the particle size of the sample as it is supposed to facilitate the following steps. Household grinder Bosch TSM6A013B was used to grind the SCG for 5 minutes with a power rating of 180 W.

**Defatting step d)**

Soxhterm was used for the defatting. In this step, 3 gr of SCG are weighed in thimbles and placed in the extractor beaker filled with petroleum ether. The extraction beaker is placed into the apparatus. The setting temperature is 135°C. After 3 hours of defatting, the thimbles containing the SCG defatted are left overnight to dry. The sample was analyzed as pre-treated.

**Microwave-assisted extraction step a)**

The defatted SCG was treated by using the Monowave 400 (Anton Paar, Austria) according to Example 3. All the samples were frozen at -20°C before subsequent treatment or analysis.

**Enzymatic assisted extraction step b)**

The enzymatic extraction was sequentially performed as described in example 7 step b)

**Results**

Antioxidant activity measured by DPPH and dry matter.

The dry matter content of the sample pre-treated by HPP was 44.17 mM Trolox eq/g with a dry matter of 7.6 %. The application of the enzymatic treatment on the pretreated HPP spent coffee ground sample increased the yield to 12.96 % and antioxidant activity was measured by DPPH of 54.26 mM Trolox eq/g. The combination of the enzymatic treatment and monowave-assisted extraction on the pre-treated HPP sample showed a yield in dry matter of 38%

**Example 10**

**High-pressure process (HPP)**

The high-pressure process (HPP) operates at low temperatures (usually up to 60 °C) and high pressures (usually 100-600 MPa) to extract the compounds quickly, requiring low quantities of organic solvents and at the same time provides retrievals similar to other extraction technologies. It is a good alternative to traditional heat treatment because it decreases the extraction time, and solvent consumption, and increases the extraction yield. The setting is of a ratio 1/1.5 with water at 600 MPa for 5 min.

**Grinding step c)**

Household grinder Bosch TSM6A013B was used to grind the SCG for 5 minutes with a power rating of 180 W.

**Defatting step d)**

Soxhterm has been used for the defatting according to the defatting step in example 9.

**Microwave -assisted extraction step a)**

SCGwas treated by using the Monowave 400 (Anton Paar, Austria) as described in Example 3.

**Enzymatic assisted extraction step b)**

The enzymatic extraction was performed as described in Example 9 in a ratio of 1:4, SCG: water.

**Results**

Antioxidant activity measured by DPPH and dry matter.

The dry matter content of the sample pre-treated by HPP was 50.59 mM Trolox eq/g with a dry matter of 5.98%. The application of the enzymatic treatment on the pretreated HPP spent coffee ground sample increased the yield to 11.44% and antioxidant activity was measured by DPPH of 52.86 mM Trolox eq/g. The combination of the enzymatic treatment and monowave-assisted extraction on the pre-treated HPP sample showed a yield in dry matter of 38%

**Chemical characterization**

The soluble matter was evaluated in all the extracts. Overall, the soluble matter ranged from 3.92 $\pm$ 0.24 to 33.53 $\pm$ 0.48 g/100g of SCG (Figure 7).

The cryogenic milling led to the increase of extracted soluble matter to 40 % in SCG-C compared to SCG (3.92 $\pm$ 0.24 g/100g of SCG) and to 30 % in SCG-CE compared to SCG-E (12.96 $\pm$ 0.22 g/100g of SCG).

**Characterization of the antioxidant dietary fiber**

Table 3 below shows the characteristics of a spray dried extract using MAE followed by EAE.

Table 3

| Characteristics | | |
|---|---|---|
| **Moisture content** | | 8.94 % ± 0.002 |
| **Ash** | | 3.58% ± 0.002 |
| **Protein** | | 7.83% ± 0.064 |
| **Caffeic acid** | | 1.42 mg/g extract ± 0.103 |
| **TPC (Folin-Ciocalteu)** | | 28.46 mg GAE/g extract ± 0.438 |
| **AOA** | DPPH | 33.65 mg TE/g extract ± 0.129 |
| | ABTS | 14.09 mg TE/g extract ± 0.513 |
| | FRAP | 97.97 mg TE/g extract ± 0.589 |
| **Mannose (HPLC)** | | 57.67 mg/g extract ± 4.352 |
| **OHC** | | 4.48g oil held/g extract ±0.121 |
| **WSI** | | 87.7% ± 0.957 |

Figure 4 shows the quantification of galactose, fucose, arabinose, galactose, glucose, mannose, and fructose in SCG after the combination of microwave extraction and enzymatic treatment (SCG-ME from an industrial coffee brewing plant.

Mannose (41,35 ± 0,79 mg/g of SCG d.m.), glucose (35,5 ± 3,28 mg/g of SCG d.m.) and galactose (23.81 ± 0,08 mg/g of SCG d.m.) were the most detected sugars in SCG-ME. As observed in a previous study (not reported here) the enzymatic treatment had a higher impact on the concentrations of sugars compared to the microwave. The presence of high concentrations of mannose and galactose in SCG-ME confirmed the breakage of the galactomannan polymers from insoluble fiber. The SCG can be considered a good source of galactomannans which consist of a linked 1,4 mannan chain with galactose ramification with different degrees of branching.

As shown by the data in the histogram of figure 8, the highest radical scavenging activity was found in SCG-ME with values of 89.21 ± 0.70 and 30.31 ± 0.18 μmol of Trolox/g of SCG in ABTS and DPPH assays, respectively, while 126.97 ± 4.14 μmol of $FeSO_4$ in FRAP assay, confirming how both microwave and enzymatic treatments increased the antioxidant properties of SCG. Indeed, SCG-M and SCG-E were the other samples with high antioxidant properties, with SCG-M with higher values in all three assays, but with no statistical differences for DPPH assay. The cryogenic milling did not increase the antioxidant properties of SCG, and no statistical differences were found between SCG and SCG-C and between SCG-E and SCG-CE.

According to the invention it was found that the benefit of the invention, in terms of amount of extracted soluble matter, is significantly higher if the EAE step is carried out following the MAE step, rather than in the opposite sequence; the following example shows the results, by way of comparison, when the MAE step is carried out following the EAE step.

**Example 11 (Comparative): Enzymatic assisted extraction followed by Microwave assisted extraction**

SCG from an industrial coffee brewing process dried to a water content of about 5% and grinded as described in the example 7 was used:

1. The SCG was defatted by petroleum ether at room temperature with a ratio of 1:4 (SCG/petroleum ether) for 16 hours and stirred at 300 rpm
2. Enzymatic extraction was performed on SCG diluted 1:4 with water. Viscozyme and Celluclast were added in concentrations of 33 ul/g and 11.3 ul/g respectively at pH 5.9 and temperature of 55°C.
3. SCG pre-treated with enzymes and supernatant was further extracted by using Monowave 400. The process was performed by using the stirrer speed at 300 rpm and the maximum power of 850 W to reach 200°C, then the temperature was maintained constant for 10 min and after decreased to 70°C.

**Analysis of dry matter**

The supernatant obtained after both treatments was separated by centrifugation at 4700xg for 10 min. Around 1 ml of supernatant was transferred onto an aluminum dish before. The weights of the sample and aluminum dish were noted carefully. The dish was dried overnight in an oven at 100 C until constant weight. The dry weight of the sample was determined and expressed as mg SDM/g SCG, taking into consideration the dilution used for the extraction process. The extraction of SCG was performed in triplicate.

| **Dry matter** | | | | | | |
|---|---|---|---|---|---|---|
| Sample | Tray | with sample | sample | after drying | dry matter | % dry matter |
| EAE+MAE1 | 1.1875 | 2.1588 | 0.9713 | 1.2454 | 0.059611 | 24.55% |
| EAE+MAE2 | 1.1696 | 2.0344 | 0.8648 | 1.216 | 0.053654 | 24.82% |
| EAE+MAE3 | 1.1787 | 2.184 | 1.0053 | 1.2412 | 0.06217 | 24.74% |

**Example 12: Microwave Assisted extraction followed by enzymatic assisted extraction**

The same SCG of example 11 was used.

1. The SCG was defatted according to the same procedure and conditions of example 11, step 1.
2. The defatted SCG, diluted 1:4 with water, was extracted by using Monowave 400 according to the procedure of example 11, step 3.
3. The SCG and supernatant from step 2 was further extracted by enzymatic assisted extraction by adding Viscozyme and Celluclast, with the same concentrations and under the same conditions of example 11, step 2.

**Analysis of dry matter**

The supernatant obtained after both treatments was separated by centrifugation at 4700xg for 10 min. Around 1 ml of supernatant was transferred onto an aluminum dish before. The weights of the sample and aluminum dish were noted carefully. The dish was dried overnight in an oven at 100 C until constant weight. The dry weight of the sample was determined and expressed as mg SDM/g SCG, taking into consideration the dilution used for the extraction process. The extraction of SCG was performed in triplicate.

| **Dry matter** | | | | | | |
|---|---|---|---|---|---|---|
| Sample | Tray | with sample | sample | after drying | dry matter | % dry matter |
| MAE1+EAE | 1.2377 | 2.4813 | 1.2436 | 1.3285 | 0.0730 | 29.21 |
| MAE2+EAE | 1.2476 | 2.6883 | 1.4407 | 1.3546 | 0.0743 | 29.73 |
| MAE3+EAE | 1.2467 | 2.8587 | 1.612 | 1.3703 | 0.0766 | 30.67 |

## Claims

**1.** A process for upcycling spent coffee grounds (SCG) into an antioxidant dietary fiber comprising the steps of:

- a) microwave assisted extraction (MAE) of the spent coffee ground material in water at a temperature up to 200 °C and for a time of at least 5 min, followed by:
- b) enzymatic assisted extraction of (EAE) the spent coffee ground material extracted according to step a) in an aqueous solution comprising an enzyme having endo-beta-glucanase and/or an enzyme having cellulase activity and

recovering the soluble matter.

2. The process of claim 1, further comprising the step of:
c) grinding the spent coffee ground material to be subjected to microwave assisted extraction to a particle size having a D[4,3] below 100 microns, preferably by cryogenic milling.

3. The process of claim 1 or 2, further comprising the step of:
d) defatting the spent coffee ground, prior to step a), to reduce the fat content below 5% wt.

4. The process according to claim 3, wherein the defatting step d) is carried out by Soxhlet extraction using petroleum ether for a time of at least 2 hours.

5. The process according to any of claims 1 to 4 comprising the step of spray drying the soluble matter from steps a) and b) to obtain said antioxidant dietary fiber in a spray dried form.

6. The process according to any of claims 1 to 5 wherein in step b) the amount of the enzyme having endo-beta-glucanase activity equivalent to 300 amyloglucosidase Units/ml is from 33 to 66 μL/g of SCG.

7. The process according to any of claims 1 to 6 wherein in step b) the amount of the enzyme having cellulase activity equivalent to 700 endo-glucanase unit/g is from 11 to 40 μL/g SCG.

8. The process according to any of claims 1 to 7 wherein the step of enzymatic assisted extraction is carried out in a temperature range of from 45 to 65 °C., at a pH of from 4. 5 to 6 for a time of from 3 to 15 hours.

9. The process according to any of the preceding claims wherein the MAE step a) is carried by applying a microwave assisted extraction to a slurry of SGC in water at a SCG/water ratio of from 1:3 to 1:4.

10. The process according to any of claims 1 to 9 wherein, prior to the MAE extraction step, the SCG is subjected to a pretreatment selected from a pulsed electric field treatment or high-pressure extraction or both.

11. The process according to claim 10, wherein the pulsed electric field treatment and/or the high pressure extraction is carried out before the grinding step c) and the defatting step d).

12. The process according to claim 10 or claim 11, wherein the pulsed electric field treatment is carried out by mixing SCG with water in a solid/liquid ratio of from 30 to 42.5 % and with 1.000 to 10.000 pulses.

13. The process according to claim 10 or claim 11, wherein the high pressure extraction is carried out at a temperature below 60 °C and at a pressure of from 100 to 600 MPa.

14. A dried antioxidant dietary fiber obtainable by a process according to any of claims 1 to 12.

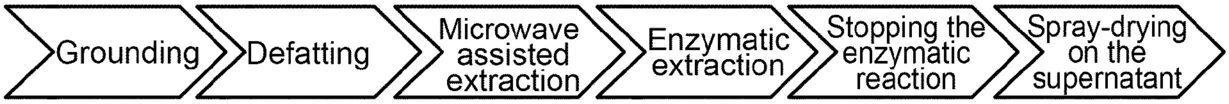


FIG.1

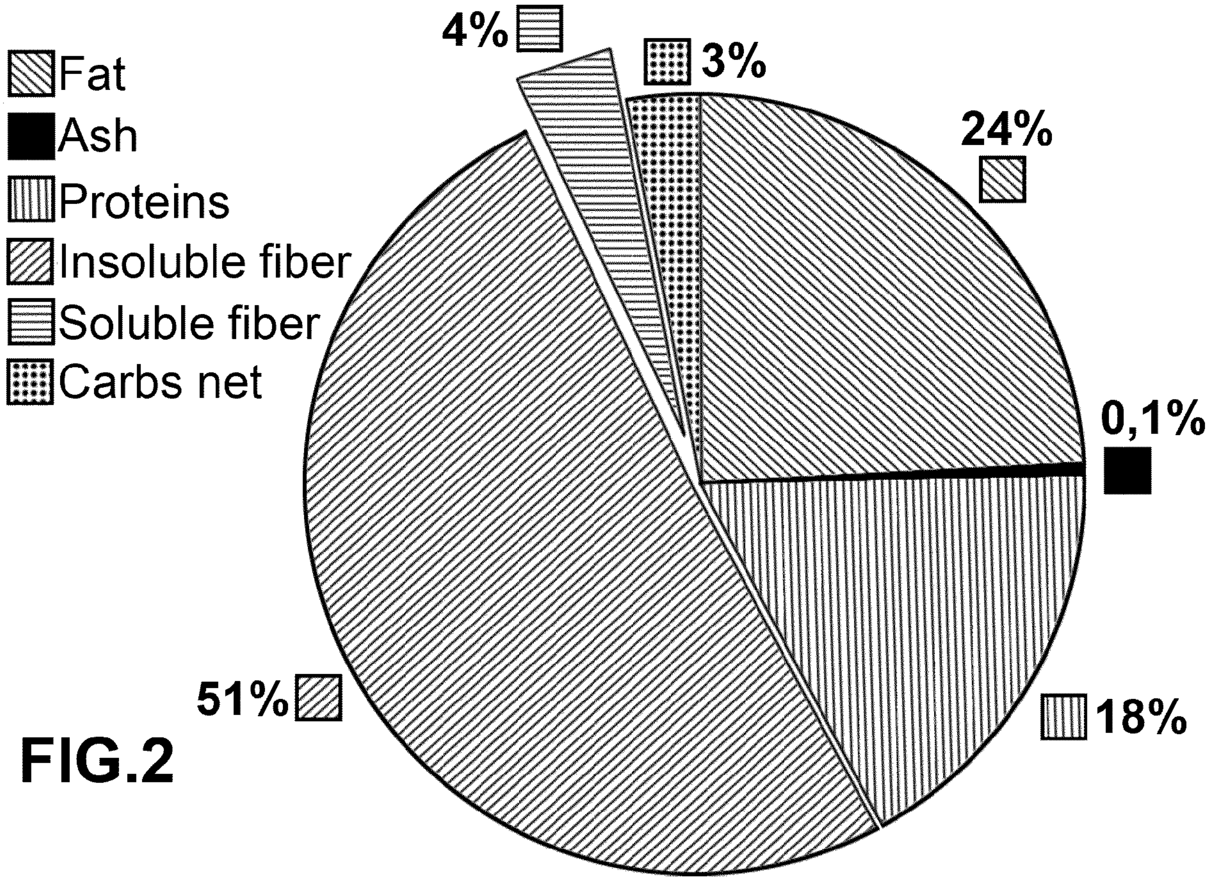


FIG.2

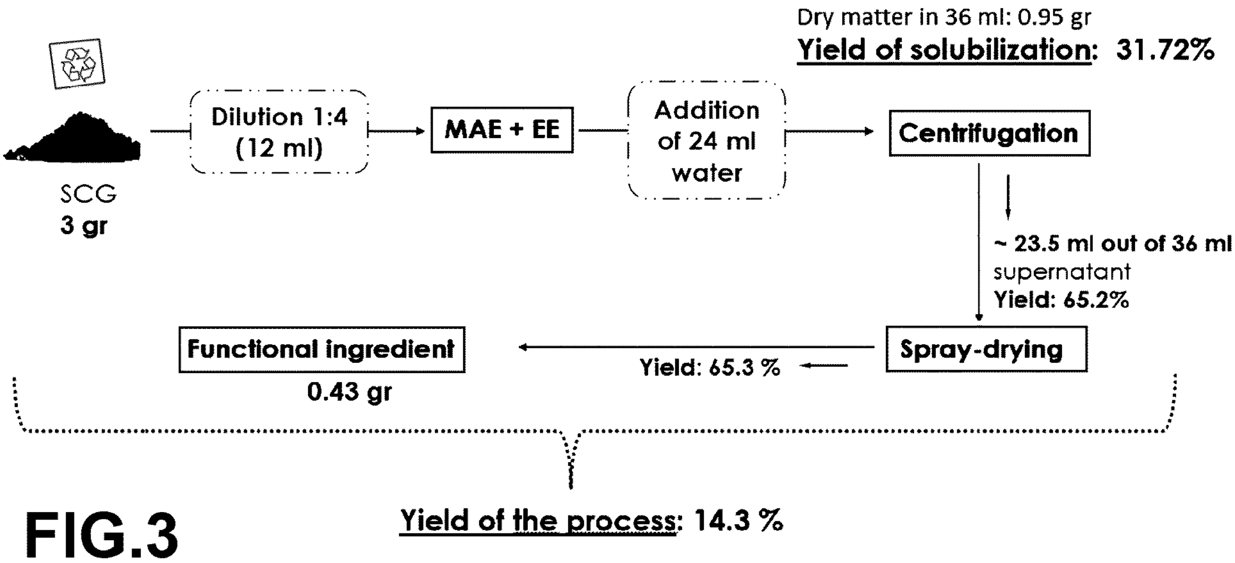


FIG.3

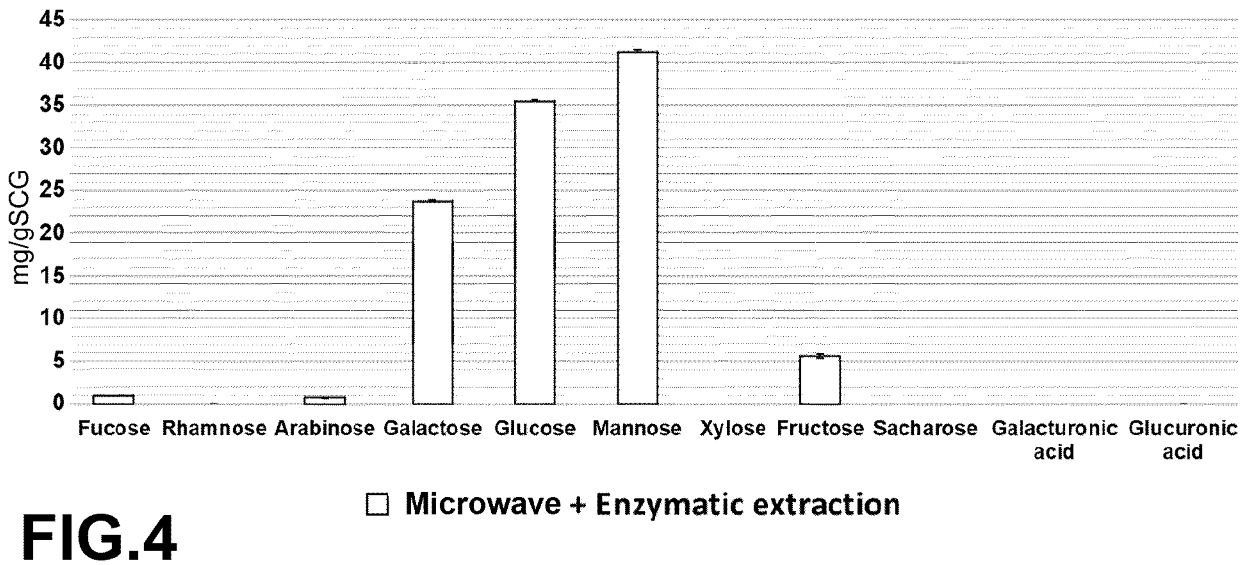
45
40
35
30
25
20
15
10
5
0
mg/gSCG
Fucose
Rhamnose
Arabinose
Galactose
Glucose
Mannose
Xylose
Fructose
Sacharose
Galacturonic acid
Glucuronic acid
Microwave + Enzymatic extraction

FIG.4

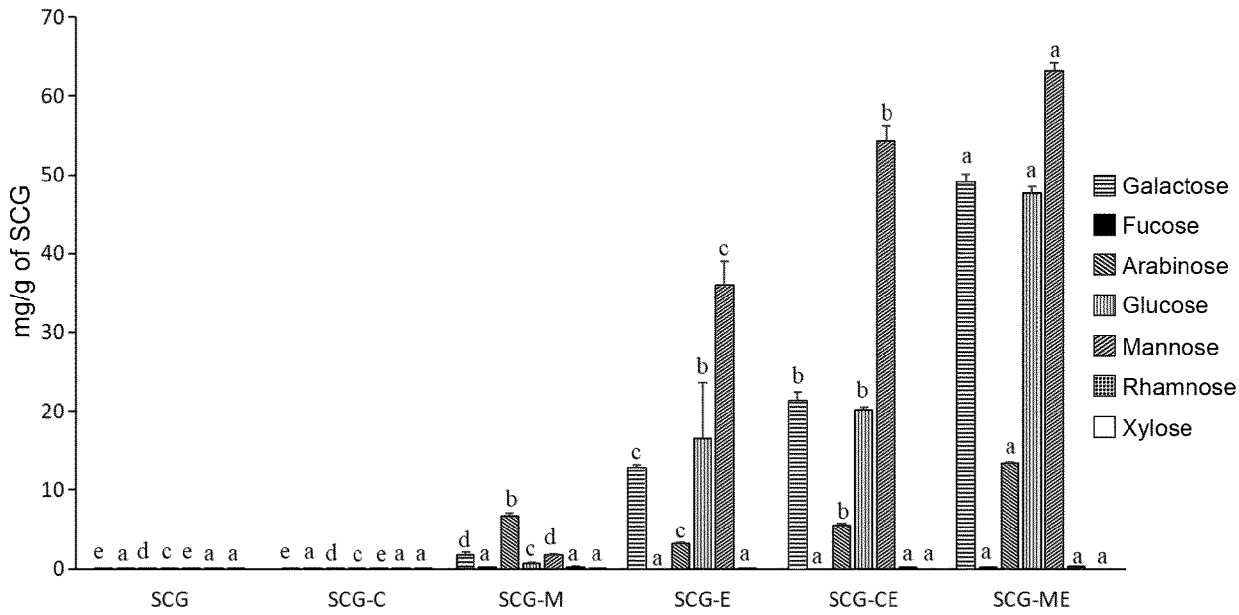
70
60
50
40
30
20
10
0
mg/g of SCG
SCG
SCG-C
SCG-M
SCG-E
SCG-CE
SCG-ME
Galactose
Fucose
Arabinose
Glucose
Mannose
Rhamnose
Xylose

FIG.5

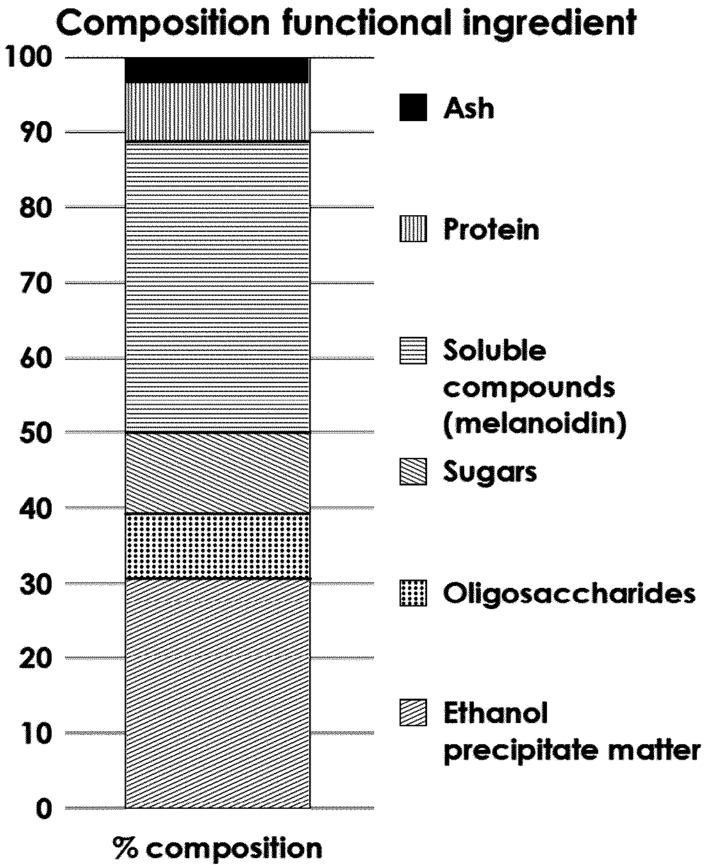


FIG.6

| Characteristics | | |
|---|---|---|
| Moisture content | | 8.94 % ± 0.002 |
| Ash | | 3.58% ± 0.002 |
| Protein | | 7.83% ± 0.064 |
| Caffeic acid | | 1.42 mg/g extract ± 0.103 |
| Caffeine | | 1.32 mg/g |
| TPC (Folin-Ciocalteu) | | 28.46 mg GAE/g extract ± 0.438 |
| AOA | DPPH | 33.65 mg TE/g extract ± 0.129 |
| | ABTS | 14.09 mg TE/g extract ± 0.513 |
| | FRAP | 97.97 mg TE/g extract ± 0.589 |
| OHC | | 4.48g oil held/g extract ±0.121 |
| WSI | | 87.7% ± 0.957 |

TABLE 2

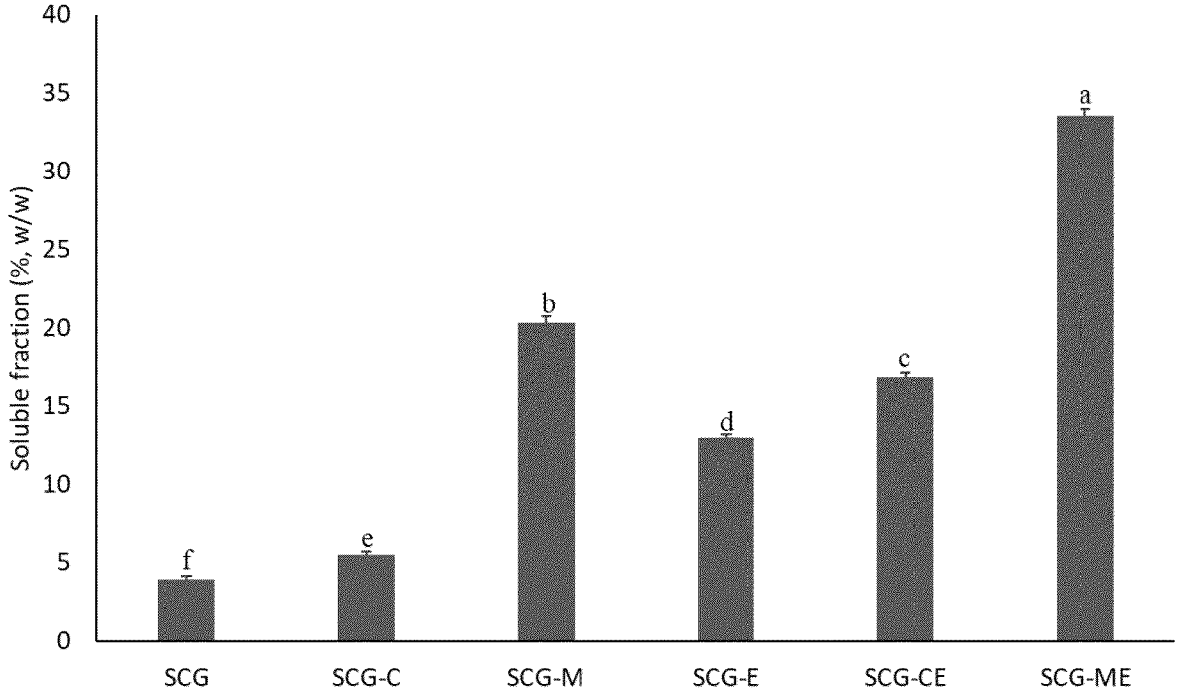
Soluble fraction (%, w/w)
40
35
30
25
20
15
10
5
0
f
e
b
d
c
a
SCG
SCG-C
SCG-M
SCG-E
SCG-CE
SCG-ME

FIG.7

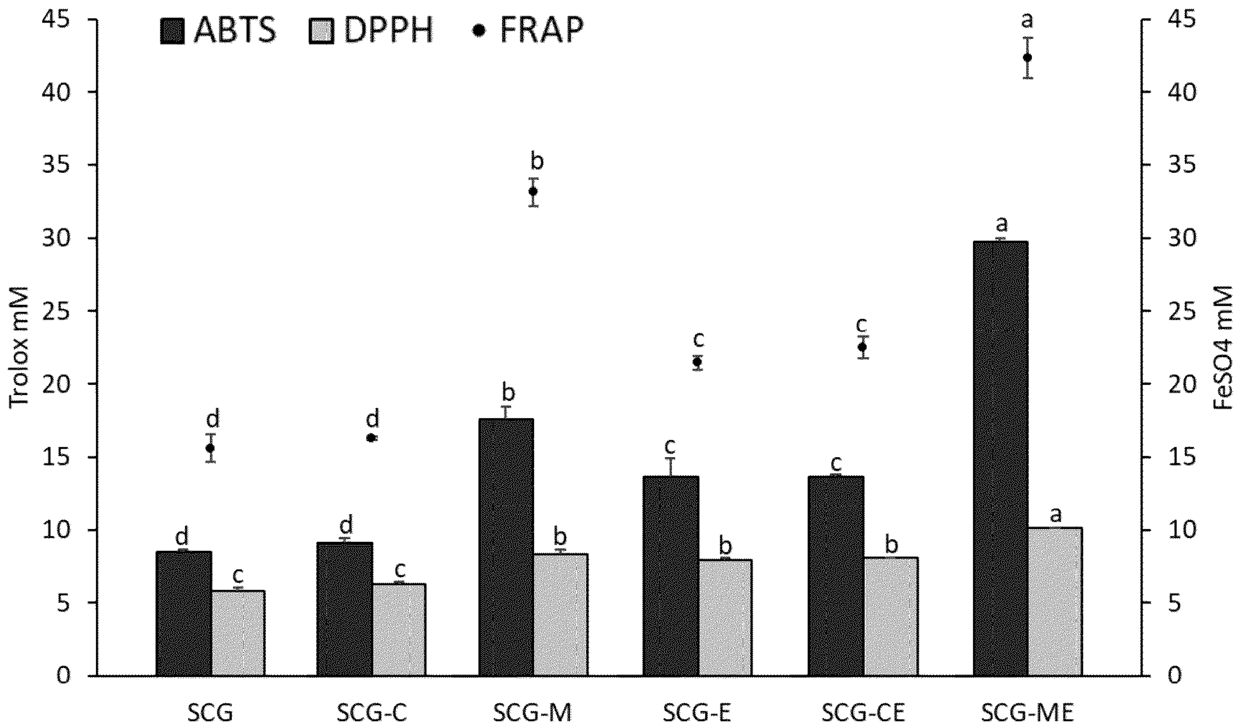
ABTS
DPPH
FRAP
Trolox mM
FeSO4 mM
45
40
35
30
25
20
15
10
5
0
SCG
SCG-C
SCG-M
SCG-E
SCG-CE
SCG-ME

FIG.8

Europäisches Patentamt
European Patent Office
Office européen des brevets

# EUROPEAN SEARCH REPORT

Application Number
EP 25 15 7347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | PASSOS CLÁUDIA P. ET AL: "Microwave superheated water extraction of polysaccharides from spent coffee grounds", CARBOHYDRATE POLYMERS, vol. 94, no. 1, 4 February 2013 (2013-02-04), pages 626-633, XP093191405, GB ISSN: 0144-8617, DOI: 10.1016/j.carbpol.2013.01.088 * 2.1. Microwave irradiation * | 1-14 | INV. A23L33/105 A23L33/21 C12P19/00 |
| A | CN 102 823 919 B (UNIV KUNMING SCIENCE & TECHNOLOGY) 28 August 2013 (2013-08-28) * example 4 * | 1-14 | |
| A | PUSHPA S MURTHY ET AL: "Recovery of Phenolic Antioxidants and Functional Compounds from Coffee Industry By-Products", FOOD AND BIOPROCESS TECHNOLOGY ; AN INTERNATIONAL JOURNAL, SPRINGER-VERLAG, NEW YORK, vol. 5, no. 3, 22 May 2010 (2010-05-22), pages 897-903, XP035025615, ISSN: 1935-5149, DOI: 10.1007/S11947-010-0363-Z * abstract * | 1-14 | TECHNICAL FIELDS SEARCHED (IPC) A23L C12P |

-/--

The present search report has been drawn up for all claims

1

| Place of search | Date of completion of the search | Examiner |
|---|---|---|
| The Hague | 2 June 2025 | Du, Mingliu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 25 15 7347

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEZER DUYGU BASKAYA ET AL: "Green processing of sour cherry (Prunus cerasus L.) pomace: process optimization for the modification of dietary fibers and property measurements", JOURNAL OF FOOD MEASUREMENT AND CHARACTERIZATION, SPRINGER US, BOSTON, vol. 15, no. 4, 22 March 2021 (2021-03-22), pages 3015-3025, XP037511881, ISSN: 2193-4126, DOI: 10.1007/S11694-021-00883-0 [retrieved on 2021-03-22] * abstract; tables 1,2 * ----- | 1-14 | |
| | | | TECHNICAL FIELDS SEARCHED (IPC) |

The present search report has been drawn up for all claims

1

| Place of search | Date of completion of the search | Examiner |
|---|---|---|
| The Hague | 2 June 2025 | Du, Mingliu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 15 7347

This annex lists the patent family members relating to the patent documents cited in the above-mentioned European search report. The members are as contained in the European Patent Office EDP file on
The European Patent Office is in no way liable for these particulars which are merely given for the purpose of information.

02-06-2025

| Patent document cited in search report | Publication date | Patent family member(s) | Publication date |
|---|---|---|---|
| CN 102823919 B | 28-08-2013 | NONE | |

EPO FORM P0459

For more details about this annex : see Official Journal of the European Patent Office, No. 12/82